(19) Europäisches Patentamt
European Patent Office
Office européen des brevets

(11) Publication number: 0 042 694 B1

(12) **EUROPEAN PATENT SPECIFICATION**

(45) Date of publication of the patent specification: **07.03.84**

(51) Int. Cl.³: **A 61 K 31/365**, C 07 H 17/08, C 12 P 19/62 // (C12P19/62, C12R1/54)

(21) Application number: **81302572.3**

(22) Date of filing: **10.06.81**

# (54) Macrolide antibiotics.

(30) Priority: **12.06.80 US 156855**

(43) Date of publication of application: **30.12.81 Bulletin 81/52**

(45) Publication of the grant of the patent: **07.03.84 Bulletin 84/10**

(84) Designated Contracting States: **AT BE CH DE FR GB IT LI LU NL SE**

(56) References cited:
**CH - A - 394 490**
**US - A - 4 161 523**

**THE JOURNAL OF ANTIBIOTICS, vol. XXIX, no. 11, November 1976, pages 1171-1181 Edit. Japan Antibiot. Res. Association TOYOKAZU KISHI et al.: "Studies on juvenimicin, a new antibiotic. II. Isolation, chemical characterization and structures"**
**CHEMICAL & PHARMACEUTICAL BULLETIN, vol. 28, no. 6, June 1980, pages 1963-1965, Edit. Pharmaceutical Society of Japan SATOSHI OMURA et al.: "Isolation and characterization of a new 16-membered lactone, protylonolide, from a mutant of tylosin producing strain, streptomyces fradiae KA-4271,2)"**

(73) Proprietor: **ELI LILLY AND COMPANY, 307, East McCarty Street, Indianapolis Indiana 46285 (US)**

(72) Inventor: **Baltz, Richard Henry, 7446 Sunset Lane, Indianapolis Indiana 46260 (US)**
Inventor: **Wild, Gene Muriel, 7455, Jewel Lane, Indianapolis Indiana 46250 (US)**
Inventor: **Seno, Eugene Thomas, Cottage No. 1, John Innes Institute Colney Lane, Norwich NR4 7UH (GB)**
Inventor: **Kirst, Herbert Andrew, 1819, Madison Village Drive Apt. B-6, Indianapolis Indiana 46227 (US)**

(74) Representative: **Crowther, Terence Roger et al, Erl Wood Manor, Windlesham Surrey GU20 6PH (GB)**

EP 0 042 694 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

# Macrolide antibiotics

This invention relates to macrolide antibiotics, in particular to compounds similar to the well-known therapeutic agent tylosin (see, for example, *Tetrahedron Letters*, 2339 (1970)).

Despite tylosin's great value, there exists a constant need to develop new antibiotics, *inter alia*, in view of the possibility of emergence of resistant strains. Further, structural modification can lead to changes in the spectrum of susceptible microorganisms. Unfortunately, chemical modification of the structure of tylosin-like macrolides has proven to be extraordinarily difficult. For instance, in *J. Org. Chem. 44 (12)*, 2050-2 (1979) there is an indication of the problems involved in the cleavage of the mycinosyl glycoside linkage of tylosin by chemical means. Indeed, in the overwhelming majority of cases, research workers in this area, intent on investigating novel structures, have been forced to search for new microorganisms, either naturally-occuring or synthetic, hoping that their cultivation will yield related structures.

Surprisingly, the Applicants have now discovered that compounds similar to tylosin but lacking tylosin's mycinosyloxy entity can be prepared by the submerged aerobic fermentation of certain *Streptomyces fradiae* strains.

According to the present invention there is provided a new macrolide antibiotic having the structural formula (I):

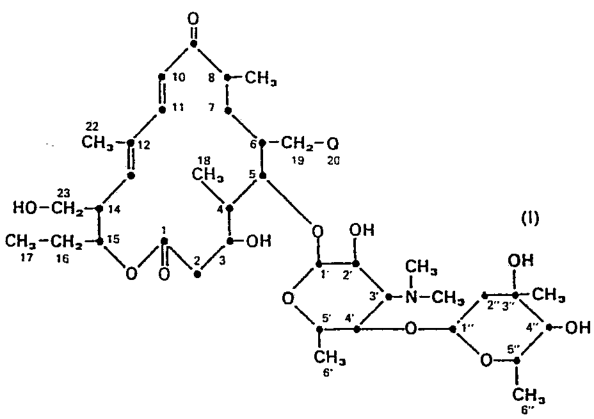

(I)

where Q is $-CH_2OH$ or $-CHO$;

or acyl esters thereof;

or acid addition salts of the compound of formula (I) or its esters.

Although no stereochemical configuration is indicated for the structure above, it is to be understood that the stereochemical configuration is identical to that of tylosin. The neutral sugar is mycarose and the amino-sugar is mycaminose.

When Q is $-CHO$, the compound of formula (I) is the new macrolide antibiotic: 23-de(mycinosyloxy)tylosin which will be called de(mycinosyloxy)tylosin or DMOT for convenience herein and which has the structure:

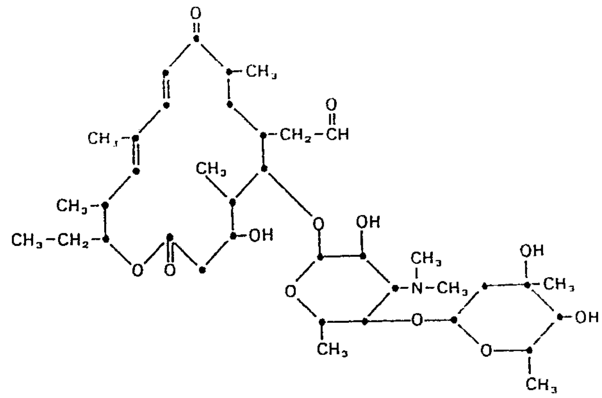

The dihydro-derivative of DMOT, i.e. 20-dihydro-23-de(mycinosyloxy)tylosin, will be called dihydro-DMOT for convenience herein, and has the structure:

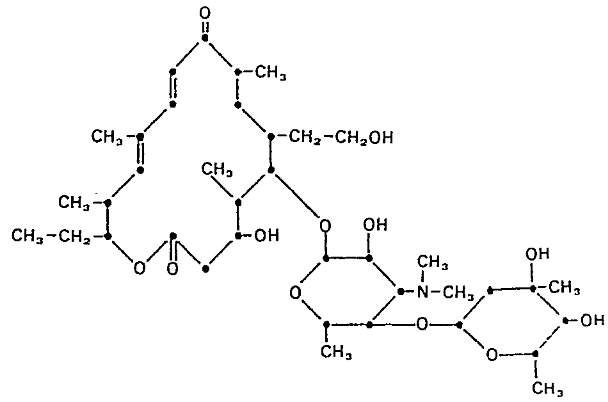

DMOT and dihydro-DMOT inhibit the growth of organisms which are pathogenic to animals. More specifically, they are antibacterial agents which are especially active against gram-positive microorganisms and *Mycoplasma* species.

The hydroxyl groups of DMOT and dihydro-DMOT can be esterified on the 2', 4'', 3'' and 3-hydroxyl groups to form useful acyl ester derivatives. In addition, dihydro-DMOT can be esterified on the 20-hydroxyl group. Esterification of the 2'-hydroxyl group is most facile. Typical esters are those of a monocarboxylic acid or hemi-esters of a dicarboxylic acid having from 2 to 18 carbon atoms.

DMOT, dihydro-DMOT and their acyl ester derivatives are basic compounds which, when treated with acids, are converted to acid addition salts. These addition salts are also part of this invention. To simplify discussions of utility, the term «DMOT compound» is used and refers to DMOT, dihydro-DMOT, a specified acyl ester derivative of these compounds, or a pharmaceutically acceptable acid addition salt of DMOT, dihydro-DMOT or of their acyl ester derivatives.

This invention further relates to a new strain of *Streptomyces fradiae*, NRRL 12171, and to the method of producing DMOT or dihydro-DMOT by culturing this strain under submerged aerobic fermentation conditions until a substantial level of

antibiotic activity is produced. DMOT or dihydro-DMOT can be extracted from basified broth filtrate with polar organic solvents and can be further purified by adsorptive or extractive procedures.

This invention also relates to a new method of preparing 23-deoxy-5-O-mycaminosyltylonolide (abbreviated herein as DOMT) and 20-dihydro-23-deoxy-5-O-mycaminosyltylonolide (dihydro-DOMT) by mild acid hydrolysis of DMOT or dihydro-DMOT, respectively. DOMT has structure:

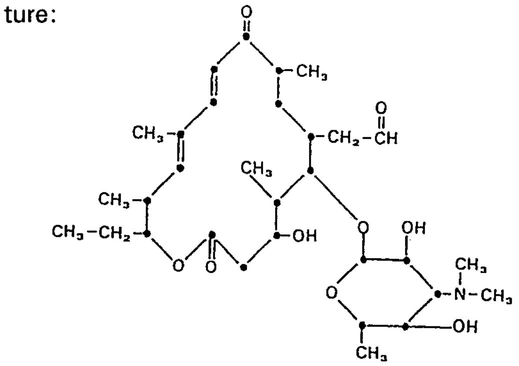

Dihydro-DMOT can be obtained by chemical reduction or by fermentation. When preparing dihydro-DMOT by chemical reduction, known procedures such as, for example, treatment with an approximately stoichiometric amount of a chemical reducing agent such as sodium borohydride in an alcoholic solvent, may be used. Dihydro-DMOT can also be produced by the *S. fradiae* NRRL 12171 of this invention under controlled fermentation conditions.

DMOT and dihydro-DMOT can be esterified at the 2', 4'', 3'' and 3-positions to give acyl ester derivatives by treatment with acylating agents using methods known in the art. In addition, dihydro-DMOT can be esterified at the 20-position. Esterification of the 2'-hydroxyl group is most facile. Typical acylating agents include anhydrides, halides (usually in combination with a base or other acid scavenger) and active esters of organic acids. Acylation can also be achieved by using a mixture of an organic acid and a dehydrating agent such as N,N'-dicyclohexylcarbodiimide. Acylations can also be carried out enzymatically as described by Okamoto et al. in U.S. Patent No. 4,092,473. Once formed, the acyl derivatives can be separated and purified by known techniques.

The 2'-monoester derivatives can be prepared by selective esterification techniques generally known in the art, such as, for example, treatment of the antibiotic with a stoichiometric quantity (or a slight excess) of an acylating agent, such as an acyl anhydride, at about room temperature for from about 1 to about 24 hours until esterification is substantially complete. The 2'-monoester can be isolated from the reaction mixture by standard procedures such as extraction, chromatography and crystallization.

Useful acyl esters are those of organic acids including aliphatic cycloaliphatic, aryl, aralkyl, heterocyclic carboxylic, sulfonic and alkoxycarbonic acids, preferably those having from 2 to 18 carbon atoms, more preferably from 2 to 12 carbon atoms.

Representative suitable esters include those derived from acids such as acetic, chloroacetic, propionic, butyric, isovaleric, alkoxycarbonic, stearic, cyclopropanecarboxylic, cyclohexanecarboxylic, β-cyclohexylpropionic, 1-adamantanecarboxylic, benzoic, phenylacetic, phenoxyacetic, mandelic and 2-thienyl-acetic acids, and alkyl-, aryl-, and aralkyl-sulfonic acids, the aryl- and aralkyl- acids optionally bearing substituents such as halogen, nitro, lower alkoxy and the like on the aromatic moiety. Suitable esters also include hemi-esters derived from dicarboxylic acids such as succinic, maleic, fumaric, malonic and phthalic acids.

Pharmaceutically acceptable ester derivatives are a preferred group. Other ester derivatives are useful, however, as intermediates.

DMOT, dihydro-DMOT and their specified acyl derivatives form acid addition salts. The acid addition salts of DMOT, dihydro-DMOT and of their acyl derivatives are also part of this invention. Such salts are useful, for example, for separating and purifying DMOT, dihydro-DMOT and their acyl derivatives. In addition the salts have an improved solubility in water.

Representative suitable salts include those salts formed by standard reactions with both organic and inorganic acids such as, for example, sulfuric, hydrochloric, phosphoric, acetic, succinic, citric, lactic, maleic, fumaric, palmitic, cholic, pamoic, mucic, D-glutamic, d-camphoric, glutaric, glycolic, phthalic, tartaric, formic, lauric, stearic, salicyclic, methanesulfonic, benzenesulfonic, sorbic, picric, benzoic, cinnamic and like acids.

Pharmaceutically acceptable acid addition salts are an especially preferred group of salts of this invention. «Pharmaceutically acceptable» salts are salts which are sufficiently non-toxic to be usable in the chemotherapy of warm-blooded animals.

This invention also relates to new methods of preparing 23-deoxy-5-O-mycaminosyltylonolide (3) (DOMT) and dihydro-DOMT by mild acid hydrolysis of DMOT and dihydro-DMOT, respectively. Mild acid hydrolysis conditions are known in the art. Appropriate solutions having a pH of about four or below can be used to accomplish the hydrolysis. Temperatures of about 20° to about 100°C can be used in this method. The reaction time needed to carry out the hydrolysis varies, depending upon the pH of the reaction mixture and the temperature used. At higher pH levels the reaction rate is slower, and at higher temperatures the reaction rate is faster. The reaction is carried out by treating either DMOT or dihydro-DMOT with a mild acid solution for a time sufficient to effect removal of the mycarosyl group to give DOMT or dihydro-DOMT, respectively.

Alternatively, and sometimes preferably, DOMT or dihydro-DOMT can be prepared by treating DMOT or dihydro-DOMT to DOMT or dihydro-DOMT, respectively. DOMT or dihydro-DOMT thus prepared can be isolated from the fermentation broth using techniques known in the art.

DOMT is identical to depoxycirramycin $A_1$ (de-epoxycirramycin $A_1$). The preparation and activity of depoxycirramycin $A_1$ are described by H. Tsukiura et al. in *J. Antibiotics 22* (3), 89-99, and 100-105 (1969). Tsukiura et al. prepare depoxycirramycin $A_1$ by treating cirramycin $A_1$ with potassium iodide in acetic acid.

Another potential method of making DOMT is suggested by T. Suzuki et al. in *Chemistry Letters 1973*, 793-798. This method involves treating antibiotic B-58941 with potassium iodide in acetic acid to obtain a product which «may be identical with depoxycirramycin $A_1$».

DOMT is also related to M-4365 $G_2$ (repromicin) and rosamicin, being 4'-hydroxy-M-4365 $G_2$ or de-epoxy-4'-hydroxy-rosamicin, respectively [see A. Kinumaki et al., *J. Antibiotics 30* (6), 450-454 (1977)]. Preparation of DOMT from either M-4365 $G_2$ or rosamicin, however, would be impractical.

DMOT and dihydro-DMOT can be prepared by culturing a strain of *Streptomyces fradiae* which produces these compounds under submerged aerobic conditions in a suitable culture medium until substantial antibiotic activity is produced. As will be appreciated by those skilled in the art, DMOT is produced first in the fermentation process. Dihydro-DMOT is produced when the fermentation is carried out for a longer time, thus permitting the DMOT present to be reduced enzymatically.

The culture medium used to grow *Streptomyces fradiae* NRRL 12171 can be any one of a number of media. For economy in production, optimal yield, and ease of product isolation, however, certain culture media are preferred. Thus, for example, preferred carbon sources in large-scale fermentation include carbohydrates such as dextrin, glucose, starch, and corn meal and oils such as soybean oil. Preferred nitrogen sources include corn meal, soybean meal, fish meal, amino acids and the like. Among the nutrient inorganic salts which can be incorporated in the culture media are the customary soluble salts capable of yielding iron, potassium, sodium, magnesium, calcium, ammonium, chloride, carbonate, sulfate, nitrate, and like ions.

Essential trace elements necessary for the growth and development of the organism should also be included in the culture medium. Such trace elements commonly occur as impurities in other constituents of the medium in amounts sufficient to meet the growth requirements of the organism. It may be necessary to add small amounts (i.e. 0.2 ml/l) of an antifoam agent such as polypropylene glycol (M.W. about 2000) to large-scale fermentation media if foaming becomes a problem.

For production of substantial quantities of DMOT or dihydro-DMOT, submerged aerobic fermentation in tanks is preferred. Small quantities of DMOT or dihydro-DMOT may be obtained by shake-flask culture. Because of the time lag in antibiotic production commonly associated with inoculation of large tanks with the spore form of the organism, it is preferable to use a vegetative inoculum. The vegetative inoculum is prepared by inoculating a small volume of culture medium with the spore form or mycelial fragments of the organism to obtain a fresh, actively growing culture of the organism. The vegetative inoculum is then transferred to a larger tank. The medium used for the vegetative inoculum can be the same as that used for larger fermentations, but other media can also be used.

*S. fradiae* NRRL 12171 can be grown at temperatures between about 10° and about 37°C. Optimum antibiotic production appears to occur at temperatures of about 28°C.

As is customary in aerobic submerged culture processes, sterile air is bubbled through the culture medium. For efficient antibiotic production the percent of air saturation for tank production should be about 30% or above (at 28°C and one atmosphere of pressure).

Antibiotic production can be followed during the fermentation by testing samples of the broth against organisms known to be sensitive to these antibiotics. One useful assay organism is *Staphylococcus aureus* ATCC 9144. The bioassay is conveniently performed by an automated turbidometric method. In addition, antibiotic production can be readily monitored by high-performance liquid chromatography with UV detection.

Following its production under submerged aerobic fermentation conditions, DMOT or dihydro-DMOT can be recovered from the fermentation medium by methods used in the fermentation art. Recovery of DMOT or dihydro-DMOT is accomplished by an initial filtration of the fermentation broth. The filtered broth can then be further purified to give the desired antibiotic. A variety of techniques may be used in this purification. A preferred technique for purification of the filtered broth involves adjusting the broth to about pH 9; extracting the broth with a suitable solvent such as ethyl acetate, amyl acetate or methyl isobutyl ketone; extracting the organic phase with an aqueous acidic solution; and precipitating the antibiotic by making the aqueous extract basic. Further purification involves the use of extraction, adsorption and/or precipitation techniques.

*The Microorganism*

The new microorganism of this invention was obtained by chemical mutagenesis of a *Streptomyces fradiae* strain which produced tylosin. The microorganism obtained by mutagenesis produces only minimal amounts of tylosin, but produces DMOT as a major component.

For characterization purposes, the new organism was compared with *Streptomyces fradiae* strain M48-E 2724.1, a tylosin-producing strain derived from *S. fradiae* NRRL 2702. *S. fradiae* NRRL 2702 was disclosed by Hamill et al. in U.S. Patent 3,178,341, issued April 13, 1965. In the discussions herein the tylosin-producing *S. fradiae* M48-E 2724.1 culture will be called «E2724.1».

The new strain which produces DMOT and dihydro-DMOT, NRRL 12171, is also classified as a strain of *Streptomyces fradiae*. In characterizing this organism, the methods recommended for the International *Streptomyces* Project for the characterization of *Streptomyces* species have been followed (E. B. Shirling and D. Gottlieb, «Methods For Characterization of *Streptomyces* Species,» *Internal. Journal of Systematic Bacteriology, 16* (3), 313-340 (1966)] along with certain supplementary tests. The following references to *S. fradiae* in the literature were consulted: 1) R. E. Buchanan and N. E. Gibbons, «Bergey's Manual of Determinative Bacteriology,» 8th ed., The Williams and Wilkins Co., Baltimore, Md., 1974, p. 815; and 2) E. B. Shirling and D. Gottlieb, «Cooperative Description of *Streptomyces*. II. Species Description from First Study,» *Internal. Journal of Systematic Bacteriology, 18* (2), 118 (1968).

The following description of the strain which produces DMOT compares its characteristics with those of the tylosin-producing *S. fradiae* strain «E2724.1».

*Characterization of the Microorganism*

The spore-chain morphology of the new strain and of the E2724.1 strain is in the Retinaculum-Apertum (RA) section. Hooks, loops, and irregular coils are short and generally not of a wide diameter. This is best observed on ISP#2 (yeast-malt extract agar) for strain E2724.1 and on Czapek's solution agar for the new strain. The spore surface is smooth; the spore shape is spherical with an average size of 0.65 μm in diameter. The diameter range is from 0.61 to 0.71 μm.

The most obvious differences between these strains are seen in their cultural characteristics. The E2724.1 strain produces aerial mycelia fairly well on most media and is in the White color series. The new strain of this invention produces very little if any aerial mycelia. When present, it is in the White to Gray color series. The reverse sides of these colonies have no distinctive pigments produced. They are light to moderate yellow in color. Melanoid pigment production is negative[1].

A summary of the important similarities and differences between the E2724.1 strain and the new strain of this invention is given in Table 1.

*Table 1*

Comparison of *Streptomyces fradiae* E2724.1 and NRRL 12171

| Similarities | Differences |
|---|---|
| Spore-chain morphology | Cultural characteristics |
| Spore-surface ornamentation | NaCl tolerance |
| Spore size | pH range |
| Lack of chromogenicity | Temperature range |
| Lack of soluble pigments | |
| Growth in selected vegetative media | |
| Starch hydrolysis | |
| Negative skim milk reaction | |
| Nitrate reduction | |
| Catalase positive | |
| Phosphatase positive | |
| Urease negative | |
| Antibiotic sensitivity pattern | |
| Carbon utilization | |
| Gelatin liquefaction | |

The morphology and growth characteristics of the *S. fradiae* E2724.1 and NRRL 12171 strains are compared in Table 2. In the tables which follow the antibiotic sensitivities (Table 3), carbon utilization (Table 4) and miscellaneous physiological characteristics (Table 5) are compared.

*Table 2*

Growth Characteristics and Morphology

| | | E2724.1 | NRRL 12171 |
|---|---|---|---|
| Sporophores | | RA | RA |
| Spore chains | | >10 | >10 |
| Spore surface[1] | | smooth | smooth |
| Spore shape | | spherical | spherical |
| ISP#2 | G[2] | good | fair |
| | R | 87. m. yellow[3] | 87. m. yellow |
| | Am | good 263. white | none |
| | Sp | none | none |
| ISP#3 | G | poor | no growth |
| | R | 263. white | — |

[1] Melanoid pigment production was tested using ISP#1 (tryptone-yeast extract broth), ISP#6 (peptone yeast extract-iron agar), ISP#7 (tyrosine agar), and ISP#7 agar without tyrosine.

*Table 2* (continued)

Growth Characteristics and Morphology

| | | E2724.1 | NRRL 12171 |
|---|---|---|---|
| | Am | poor 263. white | — |
| | Sp | none | — |
| ISP #4 | G | abundant | good |
| | R | 87. m. yellow | 87. m. yellow |
| | Am | abundant 263. white | good 92. y. white |
| | Sp | none | none |
| ISP #5 | G | good | good |
| | R | 86.1. yellow | 86.1. yellow |
| | Am | good 92. y. white | trace 93. y. gray |
| | Sp | none | none |
| ISP #7 | G | abundant | good |
| | R | 87. m. yellow | 87. m. yellow |
| | Am | abundant 263. white | good 264.1 gray |
| | Sp | none light brown | |
| Bennett's | G | poor | no growth |
| | R | 90. gy. yellow | — |
| | Am | none | — |
| | Sp | none | — |
| Ca-malate | G | good | poor |
| | R | 263. white | 92. y. white |
| | Am | good 263. white | none |
| | Sp | none | none |
| Czapek's | G | good | good |
| | R | 87. m. yellow | 87. m. yellow |
| | Am | abundant 263. white | good 92. y. white |
| | Sp | none | none |
| Glucoseasparagine | G | no growth | no growth |
| | R | — | — |
| | Am | — | — |
| | Sp | — | — |
| Tomato paste-oatmeal | G | abundant | good |
| | R | 92. y. white | 87. m. yellow |
| | Am | abundant 263. white | none |
| | Sp | none | none |

[1] Spore-surface ornamentation was determined using a scanning electron microscope.

[2] G = Growth; R = Reserve or underside of colony; Am = Aerial mycelium; Sp = Soluble pigment.

[3] Color names were assigned using the ISCC-NBS color charts (K. L. Kelly and D. B. Judd, "The ISCC-NBS Centroid Color Charts Standard Sample No. 2106," U.S. Dept. of Commerce, National Bureau of Standards, Washington, D.C. 20234).

*Table 3*

Antibiotic Sensitivity[a, b]

| Antibiotics | Conc. | Class Compound | E2724.1 | NRRL 12171 |
|---|---|---|---|---|
| Chloramphenicol | 30 μg | Nitrophenyl compound | + | + |
| Erythromycin | 15 μg | Macrolide | tr | + |
| Cephaloridine | 30 μg | β-Lactam | + | + |
| Lincomycin | 2 μg | Lincosaminide | − | − |
| Polymyxin B | 300 units | Peptide | tr | tr |
| Streptomycin | 10 μg | Aminoglycoside | + | + |
| Tetracycline | 30 μg | Tetracycline | + | + |
| Vancomycin | 30 μg | Glycopeptide | + | + |

[a] Determined by using sensitivity discs padded onto seeded-agar plates.

[b] − = resistance (no zones of inhibition), + = sensitivity (zones of inhibition), tr = trace of sensitivity.

*Table 4*

Carbon Utilization[a, b]

| Carbon Source | E2724.1 | NRRL 12171 |
|---|---|---|
| Control: no carbon | − | − |
| Control: glucose | + | + |
| L-Arabinose | − | − |
| D-Fructose | + | + |
| D-Galactose | + | + |
| i-Inositol | + | + |
| D-Mannitol | − | − |
| Raffinose | − | − |
| Salicin | − | − |
| Sucrose | + | + |
| D-Xylose | + | + |
| D-Rhamnose | − | − |

[a] − = no utilization,
+ = utilization.

[b] Determined on International *Streptomyces* Project (ISP) #9 (carbon-utilization agar) basal medium to which filter-sterilized carbon sources were added to equal a final concentration of 1.0%. Plates were incubated at 30°C and observed after 7 and 12 days.

*Table 5*

Miscellaneous Physiological Characteristics

| | E2724.1 | NRRL 12171 |
|---|---|---|
| ISP #1 (chromogenicity) | − | − |
| ISP #6 (chromogenicity) | − | − |
| ISP #7 (chromogenicity) | − | − |
| Gelatin liquefaction | − | − |
| Skim-milk reaction | − | − |
| pH growth range[1, 2] | 6.1-8.8 | 6.1-7.8 |
| Temperature growth range[1, 3] | 10-37°C | 10-30°C |
| NaCl tolerance[1, 4] | 8% | 4% |
| Starch hydrolysis[5] | + | + |
| Nitrate reduction | + | + |
| Catalase[6] | + | + |
| Phosphatase[6] | + | + |
| Urease[6] | − | − |

[1] On ISP #2 (yeast extract-malt extract agar) medium; incubated 7 days.

[2] Determined using the following buffers at a concentration of 0.05 M: citric acid, pH 3, 4, 5; 2-(N-morpholino)ethanesulfonic acid, pH 6; 3-(N-morpholino)propanesulfonic acid, pH 7; N-2-hydroxyethylpiperazine-N'-2-ethanesulfonic acid, pH 8; 2-amino-2-(hydroxymethyl)-1,3-propanediol, pH 9; 3-cyclohexylamino-1,1-propanesulfonic acid, pH 10, 11. The pH of the agar after seven days' incubation was taken as the correct value since some of the buffers failed to hold their adjusted pH. Buffer toxicity was tested by adjusting all the buffers to pH 7.0 and determining growth. No toxicity was noted.

[3] Tested at 5, 10, 15, 20, 25, 30, 37, 40, 45, 50 and 55°C.

[4] Measured by adding NaCl to the agar to equal: 0, 2, 4, 6, 8, 10 and 12% NaCl by weight.

[5] Starch hydrolysis was determined by testing for the presence of

starch with iodine on ISP#4 (inorganic salts-starch agar) plates.

[6] The methods of Blazevic and Ederer were followed for the enzyme assays (D. J. Blazevic and G. M. Ederer, "Principles of Biochemical Tests in Diagnostic Microbiology," John Wiley and Sons, New York, N.Y., 1975).

Based on the foregoing characteristics the organism which produces DMOT and dihydro-DMOT, NRRL 12171, is classified as a new strain of *Streptomyces fradiae.* This new culture has been deposited and made part of the stock culture collection of the Northern Regional Research Center, Agricultural Research, North Central Region, 1815 North University Street, Peoria, Illinois, 61604, from which it is available to the public under the accession number NRRL 12171.

As is the case with other organisms, the characteristics of *Streptomyces fradiae* NRRL 12171 are subject to variation. For example, recombinants, mutants or artificial variants of the NRRL 12171 strain may be obtained by treatment with various known physical and chemical mutagens, such as ultraviolet light, X-rays, gamma rays, and N-methyl-N'-nitro-N-nitrosoguanidine. All natural and artificial variants, mutants and recombinants of *Streptomyces fradiae* NRRL 12171 which retain the characteristic of DMOT production may be used in this invention.

*Activity of The DMOT Compounds*

The DMOT compounds inhibit the growth of pathogenic bacteria, especially gram-positive bacteria and *Mycoplasma* species. For example, Table 6 summarizes the minimal inhibitory concentrations (MIC), as measured by standard agar-dilution assays, at which DMOT (free base) inhibits certain bacteria.

*Table 6*

In Vitro Activity of DMOT Free Base

| Organism | MIC (μg/ml) |
|---|---|
| *Streptococcus pyogenes* C203 | 0.25 |
| *Streptococcus pneumoniae* Park I | 0.13 |
| *Streptococcus* sp. (Group D) 282 | 0.5 |
| *Staphylococcus aureus* 3055 | 1.0 |
| *Pasteurella multocida* | 6.25 |
| *Pasteurella hemolytica* | 25.00 |
| *Mycoplasma gallisepticum* | 0.097 |
| *Mycoplasma hyopneumoniae* | 0.39 |
| *Mycoplasma hyorhinis* | 0.78 |

The DMOT compounds have shown *in vivo* antimicrobial activity against experimental bacterial infections. When two doses of test compound were administered to mice in experimental infections, the activity observed was measured as an $ED_{50}$ value [effective dose in mg/kg to protect 50% of the test animals: see Warren Wick, et al., *J. Bacteriol.* *81*, 233-235 (1961)]. An $ED_{50}$ value observed for DMOT is given in Table 7.

*Table 7*

$ED_{50}$ Value of DMOT[a]

| Test Compound | *Streptococcus pyogenes* C203 |
|---|---|
| DMOT Free Base | 6.3 |
| Bacterial Challenge (X $LD_{50}$) | 268 |

[a] Subcutaneous; mg/kg × 2.

For the prevention or treatment of *Mycoplasma* infections in poultry, an effective non-toxic amount of a DMOT compound is administered to birds orally or parenterally. DMOT compounds are most conveniently administered with a pharmaceutically acceptable carrier, such as the water ingested by the birds.

In order to illustrate more fully the operation of this invention, the following examples are provided:

*Example 1:*

A. *Shake-flask Fermentation of DMOT*

A lyophilized pellet of *Streptomyces fradiae* NRRL 12171 is dispersed in 1-2 ml of sterilized water. A portion of this solution (0.5 ml) was used to inoculate a vegetative medium (150 ml) having the following composition:

| Ingredient | Amount (%) |
|---|---|
| Corn steep liquor | 1.0 |
| Yeast extract | 0.5 |
| Soybean grits | 0.5 |
| $CaCO_3$ | 0.3 |
| Soybean oil (crude) | 0.45 |
| Deionized water | 97.25 |

Alternatively, a vegetative culture of *S. fradiae* NRRL 12171 preserved, in 1-ml volumes, in liquid nitrogen was rapidly thawed and used to inoculate the vegetative medium. The inoculated vegetative medium was incubated in a 500-ml Erlenmeyer flask at 29°C for about 48 hours on a closed-box shaker at 300 rpm.

This incubated vegetative medium (0.5 ml) was used to inoculate 7 ml of a production medium having the following composition:

| Ingredient | Amount (%) |
|---|---|
| Beet molasses | 2.0 |
| Corn meal | 1.5 |
| Fish meal | 0.9 |
| Corn gluten | 0.9 |
| NaCl | 0.1 |
| $(NH_4)_2HPO_4$ | 0.04 |
| $CaCO_3$ | 0.2 |
| Soybean oil (crude) | 3.0 |
| Deionized water | 91.36 |

The inoculated fermentation medium was incubated in a 50-ml bottle at 29°C for about 6 days on a closed-box shaker at 300 rpm.

B. *Tank Fermentation of DMOT*

In order to provide a larger volume of inoculum, 60 ml of incubated vegetative medium, prepared in a manner similar to that described in section A, was used to inoculate 38 l of a second-stage vegetative growth medium having the following composition:

| Ingredient | Amount (%) |
|---|---|
| Corn steep liquor | 1.0 |
| Soybean oil meal | 0.5 |
| Yeast extract | 0.5 |
| $CaCO_3$ | 0.3 |
| Soybean oil (crude) | 0.5 |
| Lecithin (crude) | 0.015 |
| Water | 97.185 |

The pH was adjusted to 8.5 with 50% NaOH solution.

This second-stage vegetative medium was incubated in a 68-Liter tank for about 47 hours at 29°C.

Incubated second-stage medium (4 l) thus prepared was used to inoculate 40 liters of sterile production medium having the following composition:

| Ingredient | Amount (%) |
|---|---|
| Fish meal | 0.9188 |
| Corn meal | 1.575 |
| Corn gluten | 0.9188 |
| $CaCO_3$ | 0.210 |
| NaCl | 0.105 |
| $(NH_4)_2HPO_4$ | 0.042 |
| Beet molasses | 2.10 |
| Soybean oil (crude) | 3.15 |
| Lecithin | 0.0945 |
| Water | 90.8859 |

The pH was adjusted to 7.2 with 50% NaOH solution.

The inoculated production medium was allowed to ferment in a 68-liter tank for about 5 days at a temperature of 28°C. The fermentation medium was aerated with sterile air to keep the dissolved oxygen level between about 30% and 50% and was stirred with conventional agitators at about 300 rpm.

*Example 2:*

*Isolation of DMOT*

Fermentation broth, obtained as described in Example 1, and having a pH of 7.2, was filtered using a filter aid. Ethyl acetate (400 ml) was added to the filtrate (1450 ml). The pH of the solution was adjusted to 9.1 by the addition of sodium hydroxide. The solution was stirred 10 minutes, and the ethyl acetate separated (filtering through a filter aid to clear any emulsion which forms). The filtrate was again extracted with ethyl acetate (200 ml). Water (200 ml) was added to the combined ethyl acetate extracts and the pH of this solution adjusted to 4.1 with phosphoric acid. After extraction, the aqueous phase was separated, and the organic phase discarded. The aqueous phase was adjusted to pH 9.1 with sodium hydroxide and then concentrated to a volume of about 100 ml under vacuum. An amorphous precipitate formed. After permitting the precipitate to stand overnight, it was separated by filtration. The precipitate was dissolved in acetone (20 ml) and water (75 ml) added. The solution was concentrated under vacuum to remove acetone. The precipitate which formed was separated by filtration and washed with water to give about 500 mg of DMOT. An additional 260 mg was obtained in a similar manner from the filtrate.

*Example 3:*

*Preparation of DOMT*

DMOT (11 g), prepared as described in Example 2, was dissolved in a dilute hydrochloric acid solution (HCl added to water until the pH of the solution is 1.8). The resulting solution was allowed to stand for 24 hours at room temperature and was then adjusted to pH 9.0 by addition of sodium hydroxide. This basic solution was extracted with chloroform. The chloroform extract was dried under vaccuum to give 9.65 g of DOMT. The accompanying drawing depicts the infrared absorption spectrum of DMOT (free base) in chloroform. DMOT is a white amorphous solid which softens at about 158° and melts at about 165-167°C. Elementa analysis indicates that it has the following approximate percentage composition: carbon, 62%; hydrogen, 8%; nitrogen, 2%; oxygen, 27%. It has an empirical formula of $C_{38}H_{63}NO_{12}$ and a molecular weight of about 726 (725 as determined by mass spectrometry).

The infrared absorption spectrum of DMOT (free base) in chloroform is shown in the accompanying drawing. Observable absorption maxima occur at the following frequencies ($cm^{-1}$): 3653 (small), 3588 (shoulder), 3470 (broad), 3026 (shoulder), 2998 (shoulder), 2969 (intense), 2932 (intense), 2873 (shoulder), 1709 (intense), 1669 (medium), 1616 (v. small), 1583

(intense), 1447 (medium), 1400 (medium), 1364 (medium), 1309 (medium), 1278 (small), 1175 (medium), 1151 (medium), 1106 (small), 1066 (shoulder), 1036 (intense), 1001 (medium), 982 (medium), 972 (shoulder), 946 (small), 913 (v. small), 891 (v. small), 853 (v. small), 826 (small).

The ultraviolet absorption spectrum of DMOT in neutral ethanol exhibits an absorption maximum at 283 nm (ε 21,500).

DMOT (free base) has the following specific rotation:

$$[\alpha]_D^{25} -62.75° \text{ (c 1, } CH_3OH)$$

Electrometric titration of DMOT in 66% aqueous dimethylformamide indicates the presence of a titratable group with a $pK_a$ value of about 7.3.

DMOT free base is sparingly soluble in water, but is soluble in most polar organic solvents, such as acetone, methanol, ethanol, dimethylformamide, chloroform and dimethyl sulfoxyde. DMOT acid addition salts are more soluble in water than is DMOT base.

DMOT can be distinguished from tylosin and from DOMT by paper and thin-layer chromatography. The approximate Rf and Rx values of these antibiotics are summarized in Tables 8 and 9. In Table 8 Rx value is the ratio of movement expressed relative to that of tylosin, which was given a value of 1.0. Bioautography with *Bacillus subtilis* was used for detection.

*Table 8*

Thin-Layer Chromatography of DMOT[a]

| Compound | Rf Value | | |
|---|---|---|---|
| | A[b] | B | C |
| Tylosin | 0.53 | 0.53 | 0.67 |
| DMOT | 0.70 | 0.56 | 0.67 |
| DOMT | 0.48 | 0.17 | 0.24 |

[a] Medium: Merck, Darmstadt – Silica Gel 60.
[b] Solvent: A = ethyl acetate:diethylamine (96:4)
B = acetone:ethanol (2:1)
C = chloroform:methanol (3:1).

*Table 9*

Paper Chromatography of DMOT[a]

| Compound | Rx | |
|---|---|---|
| | D[b] | E |
| Tylosin | 1.00 | 1.00 |
| DMOT | 1.50 | 1.09 |
| DOMT | 0.50 | 0.97 |

[a] Paper: Whatman No. 1 treated with 0.75 M $KH_2PO_4$ buffer at pH 4.0 and dried.
[b] Solvent: D = ethyl acetate saturated with water
E = n-butanol saturated with water.

*Exemple 4:*

*Preparation of Dihydro-DMOT*

DMOT (50 mg), prepared as described in Example 2, was dissolved in an aqueous isopropyl alcohol solution (approximately 40%; 25 ml). Sodium borohydride (20 mg) was dissolved in a 30% aqueous isopropyl alcohol solution (10 ml). The $NaBH_4$ solution (1 ml) was added to the solution containing DMOT. The resulting mixture was stirred for 5 minutes, adjusted to pH 7.5 with phosphoric acid, and then concentrated under vacuum to remove the isopropyl alcohol. Chloroform (50 ml) was added. The pH of the aqueous phase was adjusted to 7.5. After extraction, the chloroform was separated and evaporated to dryness under vacuum to give dihydro-DMOT.

*Example 5:*

*Preparation of Dihydro-DOMT*

Dihydro-DMOT, prepared as described in Example 4, was treated in the manner described in Example 3 to give dihydro-DOMT.

*Example 6:*

*Alternative Preparation of DOMT*

DOMT was prepared from DMOT by treating DMOT in the fermentation broth in which it was produced with mild acid as described in Example 3. Isolation of DOMT was accomplished by a procedure similar to that described for DMOT in Example 2.

*Example 7:*

*2'-O-Acetyl-DMOT*

DMOT (5.0 g; 6.9 mmol) was dissolved in acetone (150 ml) an treated with 0.84 ml (8.2 mmol) acetic anhydride dropwise with stirring at room temperature. After stirring for 17 hours, solvent was evaporated under reduced pressure. The residue was dissolved in a small volume of ethyl acetate and chromatographed on silica gel (Waters Prep 500). Elution was carried out with ethyl acetate (4 liters). Fractions containing the desired product were combined and evaporated to dryness under reduced pressure to yield 4.2 g (80%) of 2'-O-acetyl-DMOT.

*Example 8:*

*2'-O-Acetyl-4''-O-phenylacetyl-DMOT*

2'-O-Acetyl-DMOT (2.7 g; 3.5 mmol) was dissolved in methylene chloride (70 ml) and pyridine (0.8 ml) and treated with a solution of 0.56 ml (3.5 mmol) phenylacetyl chloride in methylene chloride (30 ml) dropwise with stirring at room temperature. After 6 hours, solvent was evaporated and the residue was dissolved in methylene chloride and extracted with saturated $NaHCO_3$ solution. The organic layer was separated, dried ($Na_2SO_4$), filtered and evaporated. The residue was dissolved in ethyl acetate and chromatographed on silica gel (Waters Prep 500). The column was eluted with ethyl acetate (4 liters). Fractions containing the

desired product were combined and evaporated to dryness to yield 1.2 g (38%) of 2'-O-acetyl-4''-O-phenylacetyl-DMOT.

*Example 9:*

*2'-O-Acetyl-4''-O-Isovaleryl-DMOT*

In a similar manner, 1.2 g (1.6 mmol) 2'-O-acetyl-DMOT in pyridine (29 ml) was treated with 0.7 ml (3.8 mmol) isovaleryl chloride with stirring at 0°C. After working, the crude product was dissolved in toluene and chromatographed on silica gel by flash chromatography. The column was eluted with mixtures of toluene-ethyl acetate (9:1 up to 1:2), yielding 0.12 g of product. Elution of the column with methylene chloride then yielded 0.84 g (55%) of 2'-O-acetyl-4''-O-phenylacetyl-DMOT.

*Example 10:*

*4''-O-Isovaleryl-DMOT*

2'-O-Acetyl-4''-O-isovaleryl-DMOT (0.4 g; 0.47 mmol) was dissolved in 80% aqueous methanol (30 ml) and refluxed for 4.5 hours. The solution was cooled to room temperature and evaporated to dryness under reduced pressure to yield 0.13 g (34%) of 4''-O-isovaleryl-DMOT.

*Example 11:*

*4''-O-Phenylacetyl-DMOT*

2'-O-Acetyl-4''-O-phenylacetyl-DMOT (0.2 g; 0.23 mmol) was dissolved in 80% aqueous methanol (18 ml) and refluxed for 5 hours. The solution was cooled to room temperature and evaporated to dryness under reduced pressure to yield 0.16 g (86%) 4''-O-phenylacetyl-DMOT.

*Example 12:*

*2'-O-Acetyl-4''-O-Propionyl-DMOT*

2'-O-Acetyl-DMOT (1.0 g; 1.3 mmol) was dissolved in pyridine (30 ml) and treated with 0.6 ml (4.6 mmol) propionic anhydride dropwise with stirring at room temperature. After 20 hours, an additional 3.0 ml (23 mmol) propionic anhydride was added and the reaction was stirred for an additional 26 hours at room temperature. The mixture was diluted with toluene (30 ml), and solvents were evaporated under reduced pressure. The residual oil was dissolved in dichloromethane (50 ml) and extracted with saturated $NaHCO_3$ solution; the organic layer was separated, dried ($Na_2SO_4$), filtered and evaporated. The residue was loaded on a silica gel column (Waters Prep 500) and eluted with a linear gradient of toluene (4 liters) and ethyl acetate (4 liters). 305 mg of 2'-O-acetyl-3,4''-di-O-propionyl-DMOT were eluted first followed by 286 mg of 2'-O-acetyl-4''-O-propionyl-DMOT.

Each compound was refluxed in 80% aqueous methanol to yield, respectively, 4''-O-propionyl-DMOT and 3,4''-Di-O-propionyl-DMOT.

**Claims** for the Contracting States BE, CH, LI, DE, FR, GB, IT, LU, NL, SE

1. A macrolide having the structural formula (I):

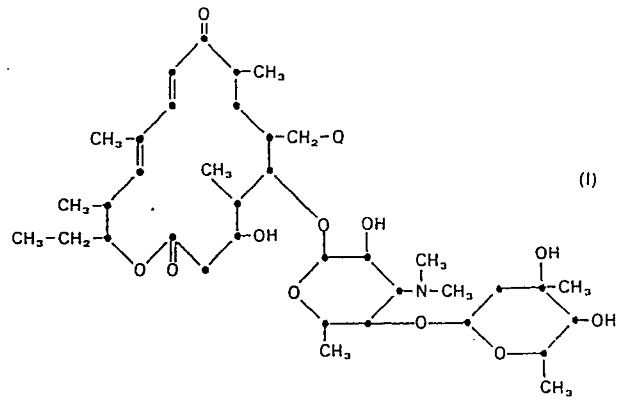

(I)

where Q is $-CH_2OH$ or $-CHO$;
or an acyl ester thereof;
or an acid addition salt of the compound of formula (I) or its esters.

2. 23-De(mycinosyloxy)tylosin.

3. 20-Dihydro-23-de(mycinosyloxy)tylosin.

4. A veterinary formulation which comprises as an active ingredient a macrolide of formula (I) as claimed in any one of claims 1 to 3, or a pharmaceutically acceptable salt or acyl ester thereof, associated with at least one suitable inert carrier therefor.

5. A process for preparing a macrolide, salt or ester as claimed in any one of claims 1 to 3, by

(a) cultivating *Streptomyces fradiae* NRRL 12171 under submerged aerobic conditions until a substantial level of antibiotic activity is produced so as to obtain 23-de(mycinosyloxy)tylosin, or

b) reducing 23-de(mycinosyloxy)tylosin with a chemical reducing agent so as to form the macrolide wherein Q is $-CH_2OH$, and

c) if it is desired to form the salt or ester, salifying or esterifying as appropriate.

6. A macrolide of formula (I) as claimed in any one of claims 1 to 3, or a pharmaceutically acceptable salt or acyl ester thereof, for use in the chemotherapy of infections caused by microorganisms which are pathogenic to warm-blooded animals.

7. A process for preparing DOMT (23-deoxy-5-O-mycaminosyltylonolide) or dihydro-DOMT by acid hydrolysis of DMOT (23-de(mycinosyloxy)tylosin) or dihydro-DMOT at a pH of 4 or less.

8. *Streptomyces fradiae* NRRL 12171.

9. A culture medium containing *Streptomyces fradiae* NRRL 12171 and assimiliable sources of carbon, nitrogen and inorganic salts.

**Claims** for the Contracting State: AT

1. A process for preparing a macrolide having the structural formula (I):

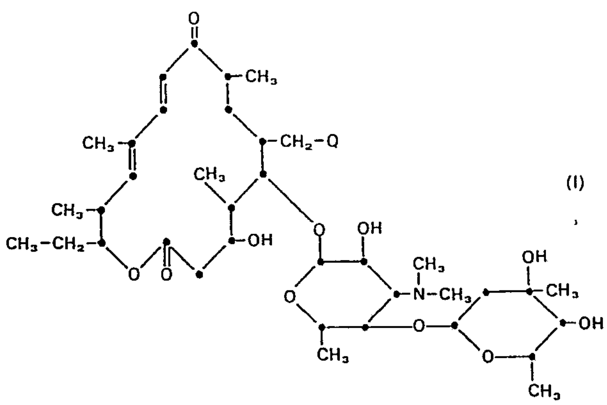

(I)

where Q is $-CH_2OH$ or $-CHO$;
or an acyl ester thereof;
or an acid addition salt of the compound of formula (I) or its esters; by

a) cultivating *Streptomyces fradiae* NRRL 12171 under submerged aerobic conditions until a substantial level of antibiotic activity is produced so as to obtain the macrolide, or

b) reducing 23-de(mycinosyloxy)tylosin with a chemical reducing agent so as to form 23-de(mycinosyloxy)tylosin wherein Q is $-CH_2CH$, and

c) if it is desired to form the salt or ester, salifying or esterifying as appropriate.

2. A process according to claim 1 for preparing 23-de(mycinosyloxy)tylosin.

3. A process according to claim 1 for preparing 20-dihydro-23-de(mycinosyloxy)tylosin.

4. A process for preparing a veterinary formulation which comprises admixing an active ingredient comprising a macrolide of formula (I) as defined in claim 1, or a pharmaceutically acceptable salt or acyl ester thereof with at least one suitable inert carrier.

5. As an antibiotic, a macrolide of formula (I) as defined in claim 1.

6. A process for preparing DOMT (23-deoxy-5-O-mycaminosyltylonolide) or dihydro-DOMT by comprising hydrolysing DMOT (23-de(mycinysyloxy)tylosin) or dihydro-DMOT at a pH of 4 or less.

**Patentansprüche** für die Vertragsstaaten BE, CH, LI, DE, FR, GB, IT, LU, NL, SE

1. Makrolid mit der Strukturformel (I)

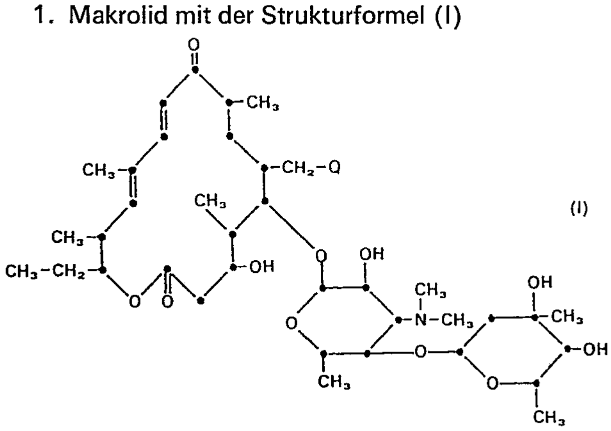

(I)

worin Q für $-CH_2OH$ oder $-CHO$ steht,
oder eines Acylesters hiervon,
oder eines Säureadditionssalzes der Verbindung der Formel (I) oder von Estern hiervon.

2. 23-De(mycinosyloxy)tylosin.

3. 20-Dihydro-23-de(mycinosyloxy)tylosin.

4. Veterinärformulierung, dadurch gekennzeichnet, dass sie ein Makrolid der Formel (I) gemäss einem der Ansprüche 1 bis 3 oder ein pharmazeutisch annehmbares Salz oder einen Acylester hiervon als Wirkstoff in Verbindung mit wenigstens einem geeigneten inerten Träger hierfür enthält.

5. Verfahren zur Herstellung eines Makrolids, Salzes oder Esters gemäss einem der Ansprüche 1 bis 3, dadurch gekennzeichnet, dass man

a) *Streptomyces fradiae* NRRL 12171 unter submersen aeroben Bedingungen bis zur Bildung einer wesentlichen Menge an antibiotischer Aktivität züchtet und so 23-De(mycinosyloxy)tylosin erzeugt oder

b) 23-De(mycinosyloxy)tylosin mit einem chemischen Reduktionsmittel reduziert und so das Makrolid bildet, worin Q für $-CH_2OH$ steht, und

c) durch Ansäuerung oder Veresterung gewünschtenfalls das Salz oder den Ester bildet.

6. Verwendung eines Makrolids der Formel (I) gemäss einem der Ansprüche 1 bis 3 oder eines pharmazeutisch annehmbaren Salzes oder eines Acylesters hiervon zur Chemotherapie von durch Mikroorganismen, die Krankheitserreger für warmblütige Tiere sind, hervorgerufenen Infektionen.

7. Verfahren zur Herstellung von DOMT (23-Deoxy-5-O-mycaminosyltylonolid) oder Dihydro-DOMT, dadurch gekennzeichnet, dass man DMOT (23-De(mycinosyloxy)tylosin) oder Dihydro-DMOT bei einem pH von 4 oder niedriger hydrolysiert.

8. *Streptomyces fradiae* NRRL 12171.

9. Kulturmedium, dadurch gekennzeichnet, dass es *Streptomyces fradiae* NRRL 12171 und assimilierbare Quellen für Kohlenstoff, Stickstoff und anorganische Salze enthält.

**Patentansprüche** für den Vertragsstaat: AT

1. Verfahren zur Herstellung eines Makrolids mit der Strukturformel (I)

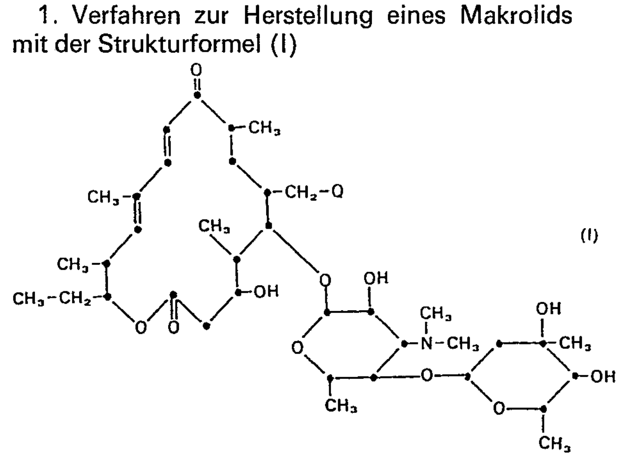

(I)

worin Q für $-CH_2OH$ oder $-CHO$ steht,

oder eines Acylesters hiervon,
oder eines Säureadditionssalzes der Verbindung der Formel (I),
oder von Estern hiervon, dadurch gekennzeichnet, dass man

a) *Streptomyces fradiae* NRRL 12171 unter submersen aeroben Bedingungen bis zur Bildung einer wesentlichen Menge an antibiotischer Aktivität züchtet und so 23-De(mycinosyloxy)tylosin erzeugt oder

b) 23-De(mycinosyloxy)tylosin mit einem chemischen Reduktionsmittel reduziert und so das Makrolid bildet, worin Q für $-CH_2OH$ steht, und

c) durch Ansäuerung oder Veresterung gewünschtenfalls das Salz oder den Ester bildet.

2. Verfahren nach Anspruch 1, dadurch gekennzeichnet, dass man 23-De(mycinosyloxy)-tylosin herstellt.

3. Verfahren nach Anspruch 1, dadurch gekennzeichnet, dass man 20-Dihydro-23-de(mycinosyloxy)tylosin herstellt.

4. Verfahren zur Herstellung einer Veterinärformulierung, dadurch gekennzeichnet, dass man einen Wirkstoff aus einem Makrolid der Formel (I) gemäss Anspruch 1 oder einem pharmazeutisch annehmbaren Salz oder Acylester hiervon mit wenigstens einem geeigneten inerten Träger vermischt.

5. Antibiotikum in Form eines Makrolids der Formel (I) gemäss Anspruch 1.

6. Verfahren zur Herstellung von DOMT (23-Deoxy-5-O-mycaminosyltylonolid) oder Dihydro-DOMT, dadurch gekennzeichnet, dass man DMOT (23-De(mycinosyloxy)tylosin) oder Dihydro-DMOT bei einem pH von 4 oder niedriger hydrolysiert.

**Revendications** pour les Etats contractants: BE, CH, LI, DE, FR, GB, IT, LU, NL, SE

1. Macrolide répondant à la formule structurale (I):

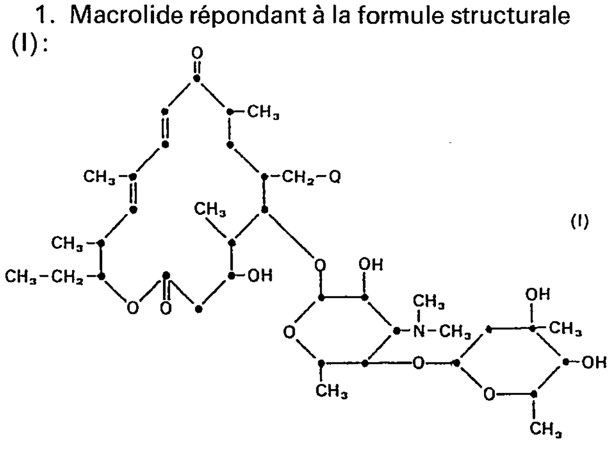

(I)

dans laquelle Q représente $-CH_2OH$ ou $-CHO$;
ou un ester acylique de ce macrolide;
ou un sel d'addition d'acide du composé de formule (I);
ou encore ses esters.

2. La 23-dé(mycinosyloxy)tylosine.

3. La 20-dihydro-23-dé(mycinosyloxy)tylosine.

4. Formulation vétérinaire comprenant, comme ingrédient actif, un macrolide de formule (I) suivant l'une quelconque des revendications 1 à 3, un de ses esters acyliques ou de ses sels pharmaceutiquement acceptables, en association avec au moins un support inerte approprié pour ce composé.

5. Procédé de préparation d'un macrolide, d'un de ses sels ou d'un de ses esters suivant l'une quelconque des revendications 1 à 3, caractérisé en ce qu'il comprend les étapes qui consistent à:

a) cultiver la souche *Streptomyces fradiae* NRRL 12171 dans des conditions aérobies submergées jusqu'à ce qu'on atteigne un important degré d'activité antibiotique afin d'obtenir la 23-dé(mycinosyloxy)tylosine, ou

b) réduire la 23-dé(mycinosyloxy)tylosine avec un agent réducteur chimique afin de former le macrolide dans lequel Q représente $-CH_2OH$, et

c) si l'on désire former le sel ou l'ester, salifier ou estérifier selon le cas.

6. Macrolide de formule (I) suivant l'une quelconque des revendications 1 à 3, un de ses esters acyliques ou un de ses sels pharmaceutiquement acceptables, en vue de l'utiliser dans la chimiothérapie des infections provoquées par les micro-organismes qui sont pathogènes vis-à-vis des animaux à sang chaud.

7. Procédé de préparation de DOMT (23-déoxy-5-O-mycaminosyl-tylonolide) ou de dihydro-DOMT par hydrolyse acide de DMOT (23-dé(mycinosyloxy)tylosine) ou de dihydro-DMOT à un pH de 4 ou moins.

8. *Streptomyces fradiae* NRRL 12171.

9. Milieu de culture contenant la souche *Streptomyces fradiae* NRRL 12171 et des sources assimilables de carbone, d'azote et de sels inorganiques.

**Revendications** pour l'Etat contractant: AT

1. Procédé de préparation d'un macrolide répondant à la formule structurale (I):

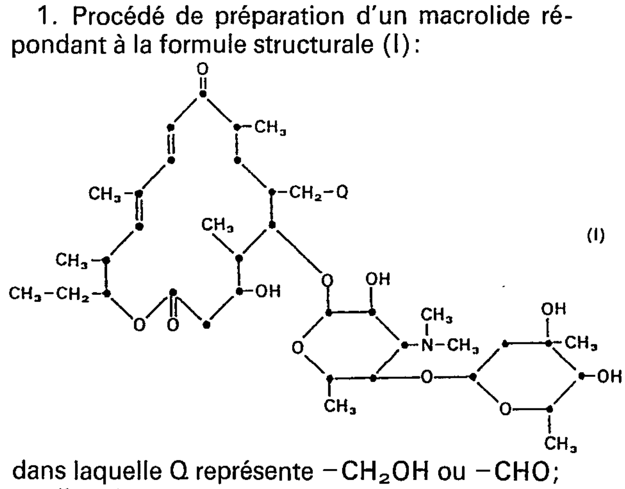

(I)

dans laquelle Q représente $-CH_2OH$ ou $-CHO$;
ou d'un de ses esters acyliques;
ou d'un sel d'addition d'acide du composé de formule (I),
ou encore de ses esters, caractérisé en ce qu'il comprend les étapes qui consistent à:

a) cultiver la souche *Streptomyces fradiae*

NRRL 12171 dans des conditions aérobies submergées jusqu'à ce qu'on atteigne un important degré d'activité antibiotique de façon à obtenir le macrolide, ou

b) réduire la 23-dé(mycinosyloxy)tylosine avec un agent réducteur chimique afin de former la 23-dé(mycinosyloxy)tylosine dans laquelle Q représente $-CH_2OH$, et

c) si l'on désire former le sel ou l'ester, salifier ou estérifier selon le cas.

2. Procédé suivant la revendication 1 pour la préparation de la 23-dé(mycinosyloxy)tylosine.

3. Procédé suivant la revendication 1 pour la préparation de la 20-dihydro-23-dé(mycinosyloxy)tylosine.

4. Procédé de préparation d'une formulation vétérinaire, caractérisé en ce qu'il consiste à mélanger un ingrédient actif comprenant un macrolide de formule (I) telle que définie dans la revendication 1, ou un ester acylique ou encore un sel pharmaceutiquement acceptable de ce macrolide avec au moins un support inerte approprié.

5. En tant qu'antibiotique, un macrolide de formule (I) telle que définie dans la revendication 1.

6. Procédé de préparation du DOMT (23-déoxy-5-O-mycaminosyl-tylonolide) ou du dihydro-DOMT, caractérisé en ce qu'il consiste à hydrolyser la DMOT (23-dé(mycinosyloxy)tylosine) ou la dihydro-DMOT à un pH de 4 ou moins.

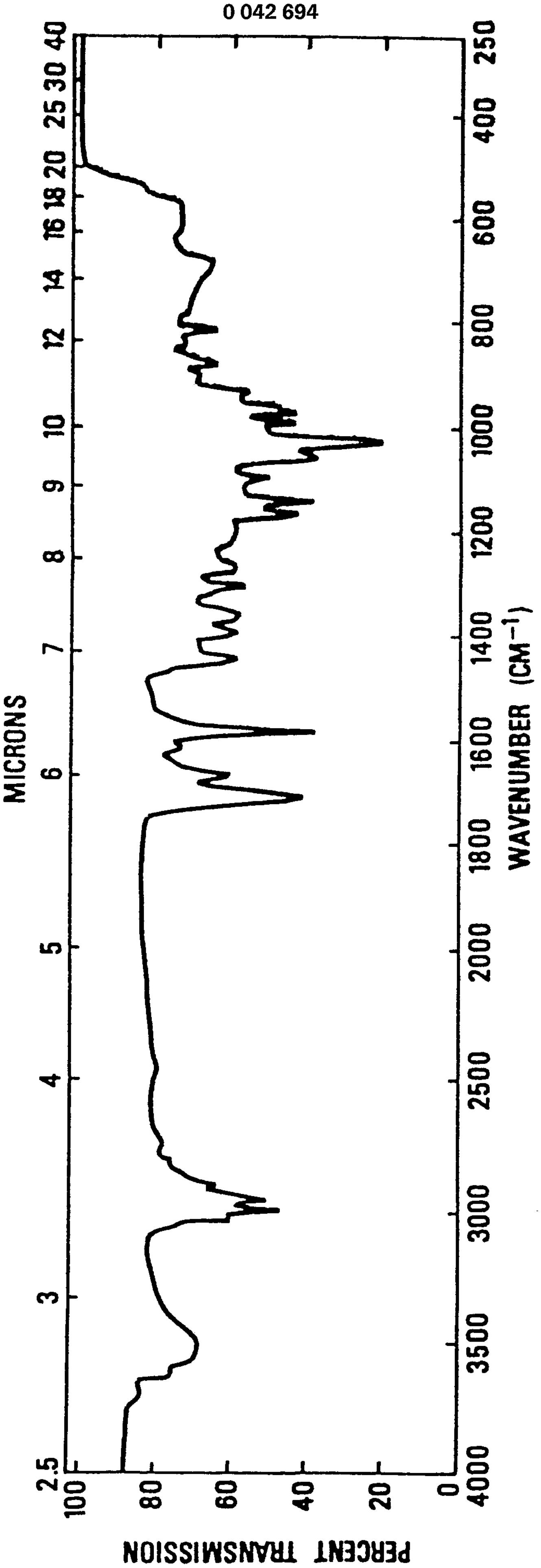
MICRONS
2.5
3
4
5
6
7
8
9
10
12
14
16 18 20 25 30 40
WAVENUMBER (CM⁻¹)
4000
3500
3000
2500
2000
1800
1600
1400
1200
1000
800
600
400
250
PERCENT TRANSMISSION
100
80
60
40
20
0